# EUROPEAN PATENT APPLICATION

(11) **EP 3 730 052 A1**
(43) Date of publication of application: **28.10.2020**
(21) Application number: 19170602.7
(22) Date of filing: 23.04.2019
(51) Int. Cl.: A61B 5/11, A61B 5/00, G16H 50/70, A61F 7/02

(54) **REMOTE NUDGING/HEAT TRIGGER APPROACH TO ASSESS SEDATION LEVEL**

(71) Applicant: Koninklijke Philips N.V., 5656 AG Eindhoven (NL)
(72) Inventor: VUPPALA, Sunil Kumar, 5656 AE Eindhoven (NL); BUSSA, Nagaraju, 5656 AE Eindhoven (NL); JOHNSON, Mark Thomas, 5656 AE Eindhoven (NL); SISODIA, Rajendra Singh, 5656 AE Eindhoven (NL); VOGTMEIER, Gereon, 5656 AE Eindhoven (NL)
(74) Representative: de Haan, Poul Erik

(57) **Abstract**

The present invention relates to sedation assessment. In order to facilitate sedation assessment in an environment of an imaging system, a sedation assessment system is provided. The sedation assessment system comprises at least one stimulus generator, a controlling device, at least one sensing device, and a sedation determination device. The controlling device is configured to generate at least one control signal including a first control signal and/or a second control signal. The at least one stimulus generator comprises a remote haptic device configured to induce, from a distance, a haptic sensation at a first impact region on a patient's skin in response to the first control signal. Additionally or alternatively, the at least one stimulus generator comprises a heating and/or cooling device attachable to a second impact region on a patient's skin and configured to heat or cool the second impact region for inducing a pain sensation in response to the second control signal. The at least one sensing device is configured to detect a patient reaction in response to the haptic sensation and/or the pain sensation. The sedation determination device is configured to determine, based on the detected patient reaction, a sedation level of the patient.

## Description

### FIELD OF THE INVENTION

The present invention relates to sedation assessment, and in particular to a sedation assessment system, to a medical imaging system including such a system, to a method for assessing a sedation level, to a computer program element, and to a computer readable medium.

### BACKGROUND OF THE INVENTION

Sedation quantification is an important area in scan disruptive events. As it is required to give optimal sedation dosage to a patient, it may be crucial to identify the sedation level of the patient. To assess the sedation status, physical touch/tapping at specific location is usually practiced, e.g. tapping at cheeks, forehead, hand, etc.

The identification of the objective sedation status may be one of the key problems for the imaging to select the right protocol with an adapted timing sequence and to identify how the sedation status is changing to decide on next steps. It may be particularly challenging to assess the sedation state in the noisy environment of a scanner system where it is also not possible to interact directly with the patient using touch.

### SUMMARY OF THE INVENTION

There may be a need to facilitate sedation assessment in an environment of an imaging system.

The object of the present invention is solved by the subject-matter of the independent claims, wherein further embodiments are incorporated in the dependent claims. It should be noted that the following described aspects of the invention apply also for the sedation assessment system, to the medical imaging system including such a system, to the method for assessing a sedation level, to the computer program element, and to the computer readable medium.

According to a first aspect of the present invention, a sedation assessment system is provided. The sedation assessment system comprises at least one stimulus generator, a controlling device, at least one sensing device, and a sedation determination device. The controlling device is configured to generate at least one control signal including a first control signal and/or a second control signal. The at least one stimulus generator comprises a remote haptic device configured to induce, from a distance, a haptic sensation at a first impact region on a patient's skin in response to the first control signal. Additionally or alternatively, the at least one stimulus generator comprises a heating and/or cooling device attachable to a second impact region on a patient's skin and configured to heat or cool the second impact region for inducing a pain sensation in response to the second control signal. The at least one sensing device is configured to detect a patient reaction in response to the haptic sensation and/or the pain sensation. The sedation determination device is configured to determine, based on the detected patient reaction, a sedation level of the patient.

In other words, it is proposed to use a remote haptic device to mimic a very tactile nudge or a touch and/or a wearable skin trigger with heating and/or cooling to induce a pain sensation. The remote haptic device may be modulated to generate a specific physical form of nudge/tap at specific locations, i.e. impact regions, at cheeks, forehead, hand, etc. The impact region to generate the localized stimulus may be determined based on the assessment description of sedation as defined in various sedation determining scales. A range of remote haptic stimuli may be used, including, but not limited to, acoustic fields (e.g. ultrasound), electromagnetic fields (e.g. light), aerodynamic fields (e.g. air flow), magnetic fields, etc. Since they are bidirectional, they can also be used for closed loop monitoring to, assess the impact of nudging, its response and compare with expected sedation level awareness.

In addition, the skin trigger with heating and/or cooling can be used instead of or in addition to the remote haptic device. The measurement may be done with the help of an actuator device that triggers patient feedback in a well-defined way. The actuator could be a heating/cooling device, which at an exact defined position generates a defined input signal to the patient, thereby leading to a reaction of the patient that is correlated to the patient's sedation level.

Patterns, such as intensity, duration, and/or repetition rate of the remote haptic device and/or the heating and/or cooling device may be modified to induce one or more haptic and/or pain sensations at any desired intensity, frequency, and/or duration. The remote haptic device and/or the heating and/or cooling device may be modulated to provide a standardized, reproducible, and quantified haptic stimulus, which may be potentially superior to the subjective touch of the clinician.

Examples of patient reactions in response to the haptic and/or pain sensations may include a movement of a body part with the first and/or second impact region, a change in a facial gesture of the patient, a change in a measured vital sign, a muscle response associated with the body part with the impact region, and a nerve response associated with the body part with the impact region. The movement of the body part and the change in the facial gesture may be detected with a camera or video based system. The vital sign may be monitored by a galvanic skin response (GSR) sensor. The muscle/nerve response may be detected by an electromyography (EMG) or electroencephalography (EEG) sensor.

The sedation level may be determined by correlating the detected patient reaction for a haptic feedback and/or a heating/cooling feedback with an anticipated response.

Accordingly, a touchless assessment of sedative status of a patient can be realized using a remote haptic device and/or a wearable skin trigger with heating and/or cooling for autonomous or semi-autonomous acquisition. The measurement of the patient feedback using a sensor, such as a camera, a GSR sensor, and/or an EEG/EMG sensor, to a haptic stimulus or heating/cooling stimulus can be done without an operator entering into the bore of a scanning machine, and thus may be beneficial for sedation assessment in a noisy environment of a scanner system, where it may not be possible to interact directly with the patient using touch. This may also be used for remote monitoring, or multiplexing of scans, where an anesthetist can evaluate multiple patients at remote location. Since the system is bidirectional, the expected response can also be simulated towards the evaluating person, e.g. assistant/anesthetist.

According to an embodiment of the present invention, the controlling device is configured to generate a sequence of at least one control signals to induce a sequence of different haptic sensations and/or different pain sensations.

According to an embodiment of the present invention, the remote haptic device comprises at least one of the following transmitters: i) a mid-air acoustic transmitter, ii) mid-air ultrasound haptic transmitter, iii) an air nozzle, iv) an electromagnetic wave transmitter, v) an electric transmitter, vi) a magnetic transmitter, vii) a radio-frequency transmitter, and viii) a superconducting transmitter.

The mid-air acoustic transmitter uses acoustic waves to create acoustic radiation pressure, which is capable of inducing a haptic sensation at any desired intensity, frequency, and/or duration. For example, by modulating an acoustic field at the range of haptic sensitivity e.g. 10-200 Hz the sound is perceived as vibration.

The mid-air ultrasound haptic transmitter may form an ultrasound beam being formed at a specific focal point and create an acoustic radiation pressure. The focused ultrasound waves cause, at a distance and in a non-invasive manner, touch sensation at any desired intensity, frequency, and/or duration.

The air nozzle may create an airflow pattern that gives rise to a jet of directed air capable of inducing the haptic sensation on the patient's skin at the impact region. Either direct compressed air methods or vortex-based methods maybe used to simulate the tactile sensation.

The electromagnetic wave transmitter, such as laser light, may be used to create a haptic effect at a distance from the source of excitation. The electromagnetic wave transmitters may be a pulsed laser based system, such as nanosecond laser, which, when applied to the skin evokes tactile sensation. For example, the electromagnetic radiation is configured to excite mechanoreceptors embedded in the patient's skin. For example, the nudging transmitter may be arranged as a laser transmitter operable at a suitable frequency.

The mentioned transmitters may be be used singly or in combination. For example, the nudging signal may be pulsed. In any of the above mentioned embodiments, the frequency of the single, the frequency of the pulses and/or intensity of the nudging signal may be held constant throughout the nudging operation but may also vary in other embodiments. In more detail, the intensity of the nudging signal may drop the closer the region of interest is to the target area. Proximity information may in addition or instead be modulated by varying a frequency of the pulsation of the nudging signal. The pulse frequency may drop the closer the distance between region of interest and target area. In a similar, manner the frequency of the ultrasound signal itself may be changed.

According to an embodiment of the present invention, the remote haptic device is configured to cause a change in temperature for the haptic sensation.

The change in temperature can change the sensitivity for the haptic trigger and also the reaction of the patient to the temperature in itself is information about the sedation level. It could be an extra device close/further away from the sensitive area of the haptic device to combine / separate the measurement with respect to temperature. This could be either a temperature actuator and/or the cooling / heating of the air that is causing the nudging effect on the skin. This give an additional parameter for testing sedation level reaction by heating up and/or cooling down the area for nudging on skin.

According to an embodiment of the present invention, the at least one sensing device is configured to detect at least one of the following patient reactions: i) a movement of a body part with the first and/or second impact region; ii) a change in a facial gesture of the patient; iii) a change in a measured vital sign; iv) a muscle response associated with the body part with the impact region, and v) a nerve response associated with the body part with the impact region.

For example, a video or camera based system may be used to monitor the movement of the body part and/or the change in a facial gesture. One or more GSR sensors may be used to detect a change in a measure vital sign. One or more EMG sensors may be used to monitor the muscle response. One or more EEG sensors may be used to monitor the nerve response.

According to an embodiment of the present invention, the at least one sensing device comprises an electromyography (EMG) sensor capable of detecting an electromyography signal indicative of a muscle response associated with a body part with the first and/or second impact region. The sedation determination device is configured to extract an EMG signal pattern associated with the muscle for the body part with the first and/or second impact region from a database. The sedation determination device is configured to determine a sedation level by comparing the detected EMG signal with the extracted EMG signal pattern.

The EMG approach may be preferred, since it is known where the remote nudging and/or heating/cooling will be directed and therefore it can be anticipated which muscle will most likely respond. For example, a nudge or a tickle on the hand will most probably activate a response in the biceps or triceps, etc. It is may also be easy to quantify the EMG, as both the stimulus, such as standardized and repeatable stimuli, and the response can be easily quantified.

According to an embodiment of the present invention, the at least one sensing device comprises an electroencephalography (EEG) sensor capable of detecting an EEG signal indicative of a nerve response associated with a body part with the first and/or second impact region. The sedation determination device is configured to extract an EEG signal pattern associated with the nerve for the body part with first and/or second impact region from a database. The sedation determination device is configured to determine a sedation level by comparing the detected EEG signal with the extracted EEG signal pattern.

The EEG approach determines the nerve response that is correlated to the patient's sedation level.

According to an embodiment of the present invention, the controlling device is configured to generate the at least one control signal automatically based on an evaluation of historical data of multiple patients or in response to an input request received through a user interface.

In an example, the at least one control signal may be determined automatically based on an evaluation of historical data of multiple patients, e.g. by using the machine learning and statistical techniques. In particular, any one of an intensity, an intensity gradient of the sequence of at least one control signals, a repetition rate, and a duration may be determined automatically based on the historical data to induce a haptic/pain sensation at any desired intensity, frequency, and/or duration.

In another example, any one or all of the modulations of the nudging and/or heating/cooling signal may be effected by the controlling device in response to commands received through the user interface.

According to an embodiment of the present invention, the user interface is configured to allow remote controlling of the controlling device and/or remote monitoring the detected patient reaction and/or the determined sedation level.

For example, the user interface may be arranged remotely away from the imaging room in connection with the controlling device and/or the sedation determination device via a physical cable or wireless, which may allow remote monitoring, or multiplexing of scans, where an anesthetist can evaluate multiple patients at remote location. Since the system is bidirectional, the expected response can also be simulated towards the evaluating person, e.g. assistant/anesthetist.

According to an embodiment of the present invention, the controlling device is configured to determine at least one of: i) an intensity, ii) an intensity gradient of the sequence of at least one control signals, iii) a repetition rate, iv) a duration to generate the at least one control signal or the sequence of at least one control signals.

Accordingly, one or more haptic/pain sensations may be induced at any desired intensity, frequency, and/or duration.

According to a second aspect of the present invention, a medical imaging system is provided. The medical imaging system comprises a medical imaging apparatus with a control logic and a sedation assessment system as described above and below. The control logic is configured to generate a scan protocol inducing the medical imaging system to perform a medical scan of a body part of the patient. The sedation assessment system is configured to determine a sedation level of the patient. The control logic is configured to determine, based on the determined sedation level, whether to continue the scan protocol and/or to modify or adapt the scan protocol to a preferred condition matching to the determined sedation level of the patient.

As mentioned, the sedation assessment system may be beneficial for a touchless assessment of sedative status in the noisy environment of the medical imaging system where it is not possible to interact directly with the patient using touch. In other words, the sedation level can be assessed without an operator entering into the bore of a scanning machine, e.g. CT scanner. This may also be used for remote monitoring, or multiplexing of scans, where an anesthetist can evaluate multiple patients at remote location. Since the system is bidirectional, the expected response can also be simulated towards the evaluating person, e.g. assistant/anesthetist. The identification of the objective sedation status may enable the imaging to select the right protocol with an adapted timing sequence and to identify how the sedation status to decide on next steps. This may also be beneficial for an autonomous imaging system, during times when no medical staff is present.

According to an embodiment of the present invention, the medical imaging apparatus is any one of: i) an X-ray imaging apparatus, ii) a magnetic resonance imaging apparatus, iii) a positron-emission tomography (PET) imaging apparatus, iv) a single-photon emission computed tomography, SPECT, imaging apparatus, iv) a computed tomography, CT, imaging apparatus, v) an ultrasound imaging apparatus, and vi) an optical imaging apparatus.

According to a third aspect of the present invention, a method is provided for assessing a sedation level. The method comprises the following steps: a) generating at least one control signal including a first control signal and/or a second control signal, b) inducing, with at least one remote haptic device, from a distance, a haptic sensation at a first impact region on a patient's skin of a body part in response to the first control signal, and/or heating or cooling, with a heating and/or cooling device attached to a second impact region on a patient's skin, for inducing a pain sensation in response to the second control signal, c) detecting a patient reaction in response to the haptic sensation and/or the pain sensation, and d) determining, based on the detected patient reaction, a sedation level of the patient.

According to a fourth aspect of the present invention, there is provided a computer program element, which, when being executed by at least one processing unit, is adapted to cause the processing unit to perform the method.

According to a fifth aspect of the present invention, there is provided a computer readable medium stored thereon the program element.

According to another aspect of the present invention, it is proposed to use a remote nudging technique and/or a heating/cooling trigger, together with sensing techniques, such as EEG or EMG, for understanding the patient status under sedation. This information may be used for further steps including continuous monitoring. A feedback system may also be provided for the patient sedation level. In the first embodiment, the nudging is based on an ultrasound beam to create an acoustic pressure modulated at a haptic patient sensitivity range. In the second embodiment a defined stimulus for the pain level at a defined body position via cooling/heating a defined area of the skin wherein the sequence of cooling and heating and the temperature range as well as the timing sequence is selected according to the sedation drug and probably also calibrated to a certain personal group factor. Accordingly, automatic assessment of sedation level is achieved.

These and other aspects of the present invention will become apparent from and be elucidated with reference to the embodiments described hereinafter.

### Definitions

The term *"user"* as used herein shall be understood as medical personnel at least partly involved in an administrative or organizational manner in the imaging procedure.

The term *"patient"* as used herein shall be seen as a person, or in veterinary settings, an animal (in particular a mammal), who is imaged.

### BRIEF DESCRIPTION OF THE DRAWINGS

Exemplary embodiments of the invention will be described in the following with reference to the following drawings:
Fig. 1 shows a sedation assessment system in a medical imaging system according to some embodiments of the present disclosure.
Fig. 2 shows a flow chart of a method to assess a sedation level.

### DETAILED DESCRIPTION OF EMBODIMENTS

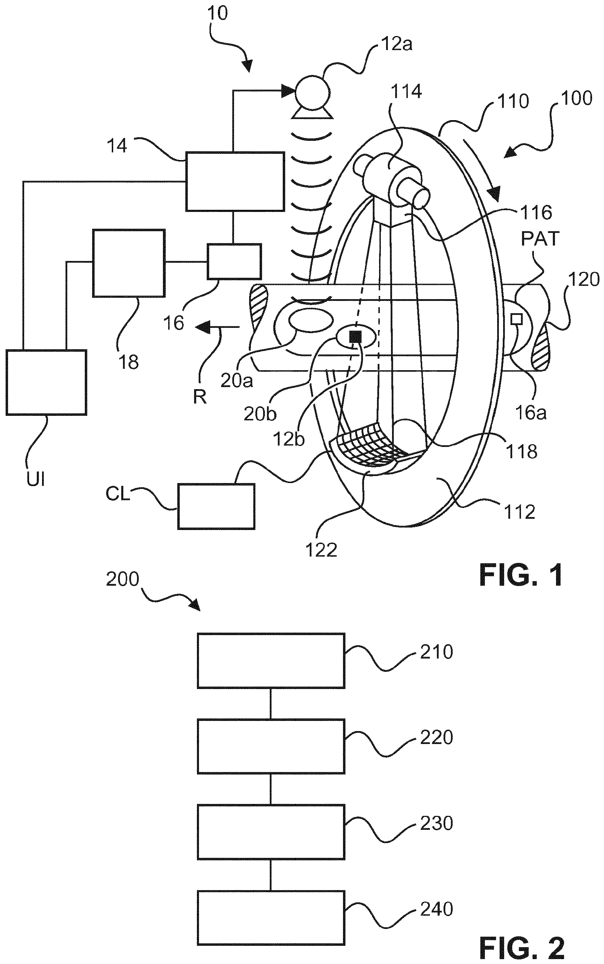

Fig. 1 shows schematically and exemplarily an embodiment of a sedation assessment system 10 in a medical imaging system 100. The medical imaging system 100 also comprises a medical imaging apparatus 110 with a control logic CL. The medical imaging apparatus 100 may be any one of an X-ray imaging apparatus, ii) a MRI apparatus, iii) a PET imaging apparatus, iv) a SPECT imaging apparatus, v) a CT imaging apparatus, vi) an ultrasound imaging apparatus, and vii) an optical imaging apparatus.

In this exemplary embodiment in Fig. 1, the medical imaging apparatus 110 is a CT scanner. The CT scanner 110 comprises a gantry 112, which is capable of rotation about a rotational axis R that extends parallel to a z direction. A radiation source 114, which in this embodiment is an x-ray tube, is mounted on the gantry 112 and is provided with a collimator 116, which forms a conical radiation beam 118 from the radiation generated by the radiation source 114. The radiation traverses a human patient PAT within a cylindrical examination zone 120. After having traversed the examination zone 120 and hence the patient PAT the radiation beam 118 is incident on a detection device 122 which comprises a two-dimensional detection surface. In addition, the detection device 122 is mounted on the gantry 112. The control logic CL is configured to generate a scan protocol inducing the medical imaging apparatus 100, in this embodiment, a CT scanner, to perform a medical scan of a body part of the patient PAT.

Sedation quantification is an important area in scan disruptive events. It is required to give optimal sedation dosage to the patients. The sedation assessment system 10 is configured to determine a sedation level of the patient within a cylindrical examination zone 120. The sedation assessment system 10 may comprise at least one stimulus generator, collectively referred to as 12, such as a remote haptic device 12a and a heating and/or cooling device 12b as illustrated in Fig. 1, a controlling device 14, at least one sensing device 16, and a sedation determination device 18.

The controlling device 14 is configured to generate at least one control signal including a first control signal and/or a second control signal. The control device 14 may be be part of, or include an Application Specific Integrated Circuit (ASIC), an electronic circuit, a processor and/or memory that execute one or more software or firmware programs, a combinational logical circuit, and/or other suitable components that provide the described functionality. The at least one control signal may be transmitted via a physical cable or wirelessly.

Optionally, the controlling device 14 may be configured to generate a sequence of at least one control signals to induce a sequence of different haptic sensations. As a further option, the controlling device is configured to determine at least one of: i) an intensity, ii) an intensity gradient of the sequence of at least one control signals, iii) a repetition rate, and iv) a duration to generate the at least one control signal or the sequence of at least one control signals.

In some embodiments, the controlling device 14 is configured to generate the at least one control signal in response to an input request received through a user interface UI. For example, any one or a combination of i) an intensity, ii) an intensity gradient of a sequence of at least one control signals, iii) a repetition rate, and/or iv) a duration may be effected by the controlling device 14 to generate the at least one control signal or the sequence of at least one control signals in response to commands received through the user interface UI. Examples of the user interface UI may include, but not limited to, a touch screen, a keystroke based interface, and a joystick arrangement. The user interface UI is preferably arranged remotely away from the imaging apparatus, for instance outside the imaging room. Alternatively, the user interface UI may be arranged inside the room. The user interface may configured to allow remote controlling of the controlling device and/or remote monitoring the detected patient reaction and/or the determined sedation level. For example, some information may be sent remotely for further evaluation by an anesthetist. The information shared to a remote person may be the haptic touch or the evaluation of the patient reaction. If the stimulus generator comprises a plurality of transmitters, such as a plurality of mid-air ultrasound transmitters, the user interface UI may include user interface elements that allow the user to select and switch between different transmitters to induce haptic/pain sensation at different impact regions. For example, in the exemplary embodiment in Fig. 1, the user may select to switch between the remote haptic device 12a and the heating and/or cooling device 12b to induce a haptic sensation at the first impact region 20a or a pain sensation at the second impact region 20b.

However, in other embodiments, the controlling device may control the at least one stimulus generator automatically. In particular, the controlling device 14 may be configured to change any one or all of the direction and modulation of the nudging signal based on historical data of the patients. For example, by using the machine learning and statistical techniques on the historical data of multiple patients, it may be defined a sequence of nudge intensity with different gradients for sensitivity control.

The at least one stimulus generator 12 may comprise a remote haptic device 12a configured to induce, from a distance, without physical contact with the patient, a haptic sensation at a first impact region 20a on a patient's skin in response to the first control signal. The remote haptic device 12a may be mounted within the imaging room or at or inside the cylindrical examination zone 120. Examples for causing such haptic sensation may include i) a mid-air acoustic transmitter, ii) a mid-air ultrasound haptic transmitter, iii) an air nozzle, iv) an electromagnetic wave transmitter, v) an electric transmitter, vi) a magnetic transmitter, vii) a radio-frequency transmitter, and viii) a superconducting transmitter.

The remote haptic device 12a may create single or multiple remote nudges with one or more transmitters at different intensities, repeat rates, and/or durations for inducing one or more different haptic sensations. The patient reactions to the one or more different haptic sensations could be any known response of a sedated patient to a conventional physical nudge, e.g. movement of the nudged body part, change in facial gestures, or change in a measured vital sign, e.g. heart rate. Optionally, the remote haptic device 12a is configured to cause a change in temperature for the haptic sensation.

Taking an ultrasound based haptic system as an example, the remote haptic device 12a may comprise one or more mid-air ultrasound haptic transmitters (not shown) configured to cause a directed or focused ultrasound beam, which can be directed to the desired impact region 20a on the patient's body. The mid-air ultrasound haptic transmitters may create an acoustic interference pattern that gives rise to the directed ultrasound beam capable of inducting the haptic sensation on the patient's skin at the impact region 20a. The focused ultrasound waves cause, at a distance and in a non-invasive manner, touch sensation at any desired intensity, frequency, and/or duration. In some embodiments, the acoustic field created by a phased array may allow forming volumetric acoustic fields, such as a palpable sphere or other geometrical configurations. The direction of the ultrasound beam maybe automatically or manually adjustable to impact a desired impact region 20a. When applied to the patient at the impact region 20a, the patient then experiences a sensation as if a physical object of the specified shape is in skin contact. Suitable acoustic shapes such as the mentioned spheres may be used to create a massage effect to gently nudge the patient towards the desired target area. It is not necessary for the patient to expose bare skin for this, because ultrasound waves are capable of coupling-in onto the skin through a suitable thin garment such as hospital gowns usually wore by patients.

Of course, other types of transmitters may also be used instead of or in additional to the mid-air ultrasound haptic transmitters. For example, in some embodiments one or more air nozzles may be used to create an airflow pattern that gives rise to a jet of directed air capable of inducing the haptic sensation on the patient's skin at the impact region 20a. Either direct compressed air methods or vortex-based methods maybe used to simulate the tactile sensation. In another example, one or more electromagnetic wave transmitters, such as laser light, may be used to create a haptic effect at a distance from the source of excitation. The electromagnetic wave transmitters may be a pulsed laser based system, such as nanosecond laser, which, when applied to the skin evokes tactile sensation. In some embodiments, heat radiation maybe used as certain receptors, e.g. TRPV1, in human skin respond not only to heat but also to pain. By eliciting both responses in turn, a haptic sensation can be caused. This may be achieved by controlling a heat source, such as a halogen lamp or other. The heat source is suitably focused using one or more reflectors and the focus is rapidly switched. The above-mentioned transmitter embodiments may be used singly or in combination or in any sub-combination.

In some embodiments, the remote haptic device is configured to cause a change in temperature for the haptic sensation. For example, it may be an extra device close or further away from the sensitive area of the haptic device to combine or separate the measurement with respect to temperature. This could be either a temperature actuator and/or the cooling / heating of the air that is causing the nudging effect on the skin. This may give an additional parameter for testing sedation level reaction by heating up and/or cooling down the area for nudging on skin, in addition to e.g. ultrasound -, laser-, and/or air-induced haptic sensation. The change in temperature may change the sensitivity for the haptic trigger and also the reaction of the patient to the temperature in itself is information about the sedation level.

Alternatively or additionally, the at least one stimulus generator 12 may comprises a heating and/or cooling device 12b, which is attachable to a second impact region 20b on the patient's skin PAT and configured to heat or cool the second impact region 20b for inducing a pain sensation in response to the second control signal. In other words, the at least one stimulus generator may be a wearable haptic device. As mentioned above, certain receptors in human skin respond not only to heat but also to pain. By eliciting both responses in turn, a haptic sensation can be caused. The sequence of cooling and heating and the temperature range as well as the timing sequence can be selected according to the sedation drug and probably also calibrated to a certain personal group factor. A gradient of the stimulus may be defined to give the input for the sensitivity control of the feedback mechanism.

The at least one sensing device 16 is configured to detect a patient reaction in response to the haptic sensation and/or the pain sensation. In some embodiments, the at least one sensing device 16 may include an optical camera or video based system. The optical camera or video-based system may be used to detect patient reactions including, but not limited to, a movement of a body part being nudged and a change in a facial gesture of the patient. The one or more video cameras may be arranged in the examination room, suitably arranged around the examination region of the medical imaging apparatus 110 to detect patient reactions. In some embodiments, the one or more video cameras may be arranged at the imaging apparatus. One or more of the cameras may be arranged inside the examination zone 120 so that the patient reactions can be detected without any structural occlusions (e.g. the gantry). In some embodiments, the at least one sensing device 16 may include one or more sensors for vital sign measurements. For example, one or more GSR sensors may be attached or wore by the patient to acquire the vital sign data of the patient whilst he/she is at or in the imaging apparatus. The vital sign measurements may be obtained during imaging or in between the imaging. In some embodiments, the at least one sensing device may comprise an EEG sensor capable of detecting an EEG signal indicative of a nerve response associated with a body part with the first and/or second impact region. In some embodiments, as illustrated in Fig. 1 the at least one sensing device may comprise one or more EMG sensors 16a capable of detecting an electromyography signal indicative of a muscle response associated with a body part with the first impact region 20a and/or second impact region 20b. As it is known where the remote nudging will be directed and/or where the heating and/or cooling device is located, it can be anticipated which muscle will most likely respond. For example, a nudge or a tickle on the hand will most probably activate a response in the biceps or triceps, etc. Thus, the one or more EMG sensors 16a can be attached to these muscles, e.g. biceps or triceps, to detect corresponding muscle response associated with the body part being nudged and/or being heated/cooled. The above-mentioned sensor embodiments may be used singly or in combination or in any sub-combination.

The sedation determination device 18 is configured to determine, based on the detected patient reaction, a sedation level of the patient. The sedation determination device 18 may be part of, or include an ASIC, an electronic circuit, a processor and/or memory that execute one or more software or firmware programs, a combinational logical circuit, and/or other suitable components that provide the described functionality.

Taking an EMG based approach as an example, as mentioned, since nudging and/or heating/cooling is directed at specific body parts, such as first impact region 20a and second impact region 20b, specific muscle response associated to that body parts, i.e. first impact region 20a and second impact region 20b, is known. The sedation determination device 18 is configured to extract an EMG signal pattern associated with the muscle for the body part with the first and/or second impact region from a database. In other words, automated EMG signal pattern associated with the muscle for the incident body parts with haptic system and/or for the body parts being heated/cooled is extracted. The sedation determination device 18 is configured to determine a sedation level by comparing the detected EMG signal with the extracted EMG signal pattern. For example, presence/absence and specific signal signature is identified and evaluation of sedation level is computed.

Other approaches, such as an EEG based approach, may also be applied. Instead of detecting specific muscle response, the EEG based approach detects specific nerve response associated to the body parts being nudged and/or being heated/cooled. The sedation determination device is configured to extract an EEG signal pattern associated with the nerve for the body part with first and/or second impact region from a database. The sedation determination device is configured to determine a sedation level by comparing the detected EEG signal with the extracted EEG signal pattern.

Reference is now made to Fig. 2 that shows a flow diagram of a method 200 to assess a sedation level. Although the below described steps relate to the above described system Fig. 1 there steps are not necessarily tied to the described system and the below described method for assessing a sedation level may also be understood as a teaching in their own right.

In step 210 at least one control signal is generated including a first control signal and/or a second control signal. Any one of an intensity, an intensity gradient of the sequence of at least one control signals, a repetition rate, and a duration may be modified to induce different haptic and/or pain sensations. In some embodiments, these parameters may be determined in response to an input request received through a user interface. In other embodiments, these parameters may be determined automatically based on an evaluation of historical data of multiple patients, e.g. by using the machine learning and statistical techniques.

In step 220, a haptic sensation is induced, with at least one remote haptic device, from a distance, at a first impact region on a patient's skin of a body part in response to the first control signal. The at least one remote haptic device may include one or more transmitters using a range of remote haptic stimuli, such as aerodynamic, acoustic, and optical stimuli, to act either on the bare skin of the patient or act indirectly through an intermediate layer such as a piece of clothes. The at least one remote haptic device may be spatially arrange at or in the imaging apparatus or is at least arranged in an imaging or treatment room where the patient is located. The at least one remote haptic device may be configured to induce single or multiple nudges at different intensities or repeat rates. Instead of or in addition to the remote haptic device, a heating and/or cooling device may be attached to a second impact region on a patient's skin, for inducing a pain sensation in response to the second control signal. The heating and/or cooling device may be wore by the patient. In some embodiments, the heating and/or cooling device may be used together with at least one remote haptic device, such as a mid-air ultrasound transmitter. The change in temperature may change the sensitivity for the haptic trigger and the reaction of the patient to the temperature in itself is information about the sedation level. This may give an additional parameter for testing sedation level reaction by heating up and/or cooling down the area for nudging on skin. The sequence of cooling and heating and the temperature range as well as the timing sequence can be selected according to the sedation drug and probably also calibrated to a certain personal group factor. A gradient of the stimulus may be defined to give the input for the sensitivity control of the feedback mechanism.

In step 230, a patient reaction is detected in response to the haptic sensation and/or the pain sensation. Examples of patient reactions include, but not limited to, a movement of a body part with the first and/or second impact region, a change in a facial gesture of the patient, a change in a measured vital sign, a muscle response associated with the body part with the impact region, and a nerve response associated with the body part with the impact region. A camera or video based system may be used to detect movements of the body part and changes in the facial gesture. A GSR sensor maybe used for vital sign readings. An EMG/EEG sensor maybe used to detect muscle/nerve responses.

In step 240, it is determined, based on the detected patient reaction, a sedation level of the patient. As nudging and/or heating/cooling is directed at specific body parts, specific patient reactions, such as movements of the body part, changes in the facial gesture, changes in a measured vital sign, and/or muscle/nerve responses, associated to the body part being nudged and/or heated/cooled is already known. The specific patient reaction pattern associated with movements of the body part, changes in the facial gesture, changes in a measured vital sign, and muscle/nerve responses for the incident body parts with haptic system or for the incident body parts with heating/cooling device may be extracted. Presence/absence and specific signal signature may thus be identified and evaluation of sedation level may be computed.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A sedation assessment system (10), comprising:
- at least one stimulus generator (12a, 12b);
- a controlling device (14);
- at least one sensing device (16, 16a); and
- a sedation determination device (18);
wherein the controlling device is configured to generate at least one control signal including a first control signal and/or a second control signal;
wherein the at least one stimulus generator comprises:
- a remote haptic device configured to induce, from a distance, a haptic sensation at a first impact region (20a) on a patient's skin (PAT) in response to the first control signal; and/or
- a heating and/or cooling device attachable to a second impact region on a patient's skin and configured to heat or cool the second impact region (20b) for inducing a pain sensation in response to the second control signal;
wherein the at least one sensing device is configured to detect a patient reaction in response to the haptic sensation and/or the pain sensation; and
wherein the sedation determination device is configured to determine, based on the detected patient reaction, a sedation level of the patient.

2. Sedation assessment system according to claim 1,
wherein the controlling device is configured to generate a sequence of at least one control signals to induce a sequence of different haptic sensations and/or different pain sensations.

3. Sedation assessment system according to claim 1 or 2,
wherein the remote haptic device comprises at least one of the following transmitters:
i) a mid-air acoustic transmitter;
ii) mid-air ultrasound haptic transmitter;
iii) an air nozzle;
iv) an electromagnetic wave transmitter;
v) an electric transmitter;
vi) a magnetic transmitter;
vii) a radio-frequency transmitter; and
viii) a superconducting transmitter.

4. Sedation assessment system according to any of the preceding claims,
wherein the remote haptic device is configured to cause a change in temperature for the haptic sensation.

5. Sedation assessment system according to any of the preceding claims,
wherein the at least one sensing device is configured to detect at least one of the following patient reactions:
i) a movement of a body part with the first and/or second impact region;
ii) a change in a facial gesture of the patient;
iii) a change in a measured vital sign;
iv) a muscle response associated with the body part with the impact region; and
v) a nerve response associated with the body part with the impact region.

6. Sedation assessment system according to any of the preceding claims,
wherein the at least one sensing device comprises an electromyography, EMG, sensor capable of detecting an EMG signal indicative of a muscle response associated with a body part with the first and/or second impact region;
wherein the sedation determination device is configured to extract an electromyography signal pattern associated with the muscle for the body part with the first and/or second impact region from a database; and
wherein the sedation determination device is configured to determine a sedation level by comparing the detected electromyography signal with the extracted electromyography signal pattern.

7. Sedation assessment system according to any of the preceding claims,
wherein the at least one sensing device comprises an electroencephalography, EEG, sensor capable of detecting an EEG signal indicative of a nerve response associated with a body part with the first and/or second impact region;
wherein the sedation determination device is configured to extract an EEG signal pattern associated with the nerve for the body part with first and/or second impact region from a database;
wherein the sedation determination device is configured to determine a sedation level by comparing the detected EEG signal with the extracted EEG signal pattern.

8. Sedation assessment system according to any of the preceding claims,
wherein the controlling device is configured to generate the at least one control signal automatically based on an evaluation of historical data of multiple patients or in response to an input request received through a user interface.

9. Sedation assessment system according to claim 8,
wherein the user interface is configured to allow remote controlling of the controlling device and/or remote monitoring the detected patient reaction and/or the determined sedation level.

10. Sedation assessment system according to any of the preceding claims,
wherein the controlling device is configured to determine at least one of: i) an intensity, ii) an intensity gradient of the sequence of at least one control signals, iii) a repetition rate, and iv) a duration to generate the at least one control signal or the sequence of at least one control signals.

11. A medical imaging system (100), comprising;
- a medical imaging apparatus (110) with a control logic (CL); and
- a sedation assessment system according to any of the preceding claims 1 to 10;
wherein the control logic is configured to generate a scan protocol inducing the medical imaging apparatus to perform a medical scan of a body part of the patient;
wherein the sedation assessment system is configured to determine a sedation level of the patient; and
wherein the control logic is configured to determine, based on the determined sedation level, whether to continue the scan protocol and/or to modify or adapt the scan protocol to a preferred condition matching to the determined sedation level of the patient.

12. Medical imaging system according to claim 11,
wherein the medical imaging apparatus is any one of:
i) an X-ray imaging apparatus;
ii) a magnetic resonance imaging apparatus;
iii) a positron-emission tomography, PET, imaging apparatus;
iv) a single-photon emission computed tomography, SPECT, imaging apparatus;
v) a computed tomography, CT, imaging apparatus;
vi) an ultrasound imaging apparatus; and
vii) an optical imaging apparatus.

13. A method (200) for assessing a sedation level, comprising:
a) generating (210) at least one control signal including a first control signal and/or a second control signal;
b) inducing (220), with at least one remote haptic device, from a distance, a haptic sensation at a first impact region on a patient's skin of a body part in response to the first control signal; and/or
heating or cooling, with a heating and/or cooling device attached to a second impact region on a patient's skin, for inducing a pain sensation in response to the second control signal;
c) detecting (230) a patient reaction in response to the haptic sensation and/or the pain sensation; and
d) determining (240), based on the detected patient reaction, a sedation level of the patient.

14. A computer program element, which, when being executed by at least one processing unit, is adapted to cause the processing unit to perform the method as per claim 13.

15. A computer readable medium having stored thereon the program element of claim 14.
